# EUROPEAN PATENT APPLICATION

(11) **EP 4 699 538 A1**
(43) Date of publication of application: **25.02.2026**
(21) Application number: 24810941.5
(22) Date of filing: 13.05.2024
(51) Int. Cl.: A61B 5/37, A61B 5/293

(54) **FUNCTIONAL DEVICE, GUIDING CATHETER, BIOLOGICAL INTERFACE SYSTEM, AND METHOD FOR INDWELLING FUNCTIONAL DEVICE**

(30) Priority: 22.05.2023 JP 2023083931
(71) Applicant: The University of Osaka, Osaka 565-0871 (JP)
(72) Inventor: SEKITANI, Tsuyoshi, Suita-shi, Osaka 565-0871 (JP); UEMURA, Takafumi, Suita-shi, Osaka 565-0871 (JP); ARAKI, Teppei, Suita-shi, Osaka 565-0871 (JP); NEZU, Toshikazu, Suita-shi, Osaka 565-0871 (JP); NAKAMURA, Hajime, Suita-shi, Osaka 565-0871 (JP); YANAGISAWA, Takufumi, Suita-shi, Osaka 565-0871 (JP)
(74) Representative: Appleyard Lees IP LLP
(86) International application number: PCT/JP2024/017585
(87) International publication number: WO 2024/241934

(57) **Abstract**

Provided is a functional device that enables at least one among measuring the activity of tissue of a living body with high resolution and applying fine stimulation to tissue of a living body, while being minimally invasive. This functional device 1 is indwelled inside a blood vessel of a living body and performs at least one among measurement of the activity of tissue outside the blood vessel and stimulation to the tissue. The functional device 1 comprises: a sheet-like flexible substrate 3; at least one functional part formed on the substrate 3; and a wiring 12 formed on the substrate 3 and connected to the functional part, wherein at least one among at least a portion of the surface of the substrate 3 and at least a portion of the surface of the functional part is covered with an antithrombotic material.

## Description

### TECHNICAL FIELD

The present invention relates to a functional device for measuring biosignals such as an electroencephalogram, a functional device for applying stimulation to blood vessels in a living body such as cerebral veins, and the like.

### BACKGROUND ART

As a technology for measuring activity of tissue of a living body, there is a technology in which an electrode is indwelled in a blood vessel. Patent Document 1 discloses a technology in which an intravascular device including an ultrathin wire made of stainless steel as a core material is used as an electrode.

### Citation List

### Patent Document

Patent Document 1: Japanese Unexamined Patent Application, Publication No. 2022-121975

### DISCLOSURE OF THE INVENTION

### Problems to be Solved by the Invention

In the conventional technology, a metal wire is used as the core material constituting the electrode. The conventional technology is highly invasive to a living body in that a steel material is indwelled inside a blood vessel.

In order to reduce the invasiveness to the living body, it is conceivable to dispose the electrode outside the cranium. However, in a case where the electrode is disposed outside the cranium, a distance from a biosignal generating portion is increased, which makes it difficult to measure the activity of the tissue of the living body with high resolution.

As described above, in the conventional technology, it is difficult to achieve both reducing the invasiveness to the living body and measuring the activity of the tissue of the living body with high resolution.

In addition, in the conventional technology, it is difficult to apply the fine stimulation to the tissue of the living body while reducing the invasiveness to the living body.

The present invention has an object to provide a functional device that enables at least one selected from measuring activity of tissue of a living body with high resolution and applying fine stimulation to the tissue of the living body while maintaining low invasiveness.

### Means for Solving the Problems

A functional device of the present invention is a functional device that is indwelled inside a blood vessel of a living body and performs at least one selected from measuring activity of tissue outside the blood vessel and applying stimulation to the tissue, the functional device including a sheet-shaped flexible substrate, at least one functional part formed on the substrate, and a wiring formed on the substrate and connected to the functional part, in which at least any one of at least a portion of a surface of the substrate and at least a portion of a surface of the functional part is covered with an antithrombotic material.

### Effects of the Invention

According to the present invention, it is possible to provide a functional device that enables at least one selected from measuring activity of tissue of a living body with high resolution and applying fine stimulation to the tissue of the living body while maintaining low invasiveness.

### BRIEF DESCRIPTION OF THE DRAWINGS

FIG. 1A is a diagram illustrating a functional device of the present invention;
FIG. 1B is a diagram illustrating a form when the functional device of the present invention is indwelled inside a blood vessel;
FIG. 1C is a diagram illustrating a portion of a functional device of another example;
FIG. 2A is a diagram illustrating an overview of a brain;
FIG. 2B is an enlarged view of a portion of FIG. 2A;
FIG. 3A is a diagram illustrating an example of a functional device;
FIG. 3B is an enlarged view of a portion of FIG. 3A;
FIG. 3C is an enlarged view of a portion of FIG. 3A;
FIG. 4A is a cross-sectional view of the functional device;
FIG. 4B is a cross-sectional view of a functional device having another configuration;
FIG. 5 is a diagram illustrating a transportation device;
FIG. 6A is a diagram illustrating a state in which a guiding catheter is inserted into the body from outside the body;
FIG. 6B is a diagram illustrating the guiding catheter and a guidewire inside a blood vessel;
FIG. 6C is a diagram illustrating the functional device inside the blood vessel;
FIG. 7A is a diagram illustrating a portion of a biological interface system;
FIG. 7B is a diagram illustrating a portion of the biological interface system;
FIG. 7C is a diagram illustrating the entire biological interface system;
FIG. 8 is a block diagram illustrating a circuit arrangement in the biological interface system;
FIG. 9A is a diagram illustrating a modified example of a functional device of the present invention; and
FIG. 9B is a diagram illustrating a form when the functional device illustrated in FIG. 9A is indwelled inside a blood vessel.

### PREFERRED MODE FOR CARRYING OUT THE INVENTION

The significance and the like of the present invention will be described before describing configurations of a functional device and the like of the present invention. A case will be described below as an example where tissue measured by a functional device is tissue around superficial cerebral veins. The tissue to be measured of a living body is not limited to the above-described tissue. The tissue to be measured may be cerebral blood vessels (for example, a cerebral venous sinus, a cerebral deep vein, and a cerebral artery) other than the superficial cerebral veins and peripheral tissue thereof, or tissue (a subdural space, a cerebrospinal space, a cerebral ventricle, a cerebral aqueduct, a cerebral cistern, visceral organs, or the like) other than the blood vessels.

As technologies including the measurement for the brain, there are known a non-invasive brain machine interface (BMI) and an invasive BMI.

For the non-invasive BMI, a wearable device or the like is used. Examples of the wearable device include a headgear device, and a device of a type which is attached to the scalp. The non-invasive BMI does not require a craniotomy. Therefore, the non-invasive BMI can be easily applied to a test subject or the like.

The electric activity of the brain is known as an electroencephalogram (EEG). The electroencephalogram is used in the medical field, and is generally used even in the fields other than the medical field. The technology using the electroencephalogram in the fields other than the medical field is referred to as brain tech.

The non-invasive BMI is non-invasive, and therefore, is characterized by the ease thereof. However, it is difficult to examine high-level brain activity in detail using the non-invasive BMI. This is because the spatial resolution and the temporal resolution are not sufficient for the non-invasive BMI. This is due to the fact that a distance from a biosignal generating part is increased and the cranium has a high electric resistance. The cranium acts as a shield when the electroencephalogram is measured.

In this way, the non-invasive BMI has a problem in that when the advanced measurement of the brain activity is made, the precision of the measurement is low.

For the invasive BMI, an implantable device or the like is used. The invasive BMI includes indwelling a device for measurement inside the cranium. Therefore, the invasive BMI enables measurement with high resolution and high precision.

The invasive BMI enables communication with a patient with amyotrophic lateral sclerosis (ALS). The invasive BMI enables a patient with amyotrophic lateral sclerosis (ALS) to highly manipulate prosthetic hands.

On the other hand, the invasive BMI includes implanting the implantable device underneath the dura mater inside the cranium. Therefore, the invasive BMI requires a craniotomy. The invasive BMI imposes significant burdens on a test subject in mental physical aspects. Due to significant burdens on a test subject, the use of the invasive BMI is extremely limited.

In this way, in the existing BMI technology, the low invasiveness of measuring an electroencephalogram via the cranium and the high-precision measurement having high temporal and spatial resolution are in a contradictory relationship. The present invention achieves these contradictory characteristics at the same time. That is, the present invention enables achievement of high measurement precision with low invasiveness.

The functional device of the present invention is flexible and has an extremely small diameter. The functional device is softer than the blood vessel and is fine enough to be disposed inside the blood vessel. Therefore, the functional device can access the brain via the blood vessels distributed throughout the body. As a result, the functional device does not require a craniotomy for indwelling the functional device in the brain. The functional device of the present invention can be said to be a functional device with extremely low invasiveness.

The greatest risk when an artifact is indwelled in the blood vessel and when the artifact accesses the brain from inside the blood vessel and is indwelled at a specified position is vascular occlusion due to thrombi. In particular, in an arterial blood vessel, the risk of vascular occlusion is high. The arterial blood vessel is fine on the downstream side, making it easy for the thrombi to cause clogging of the blood vessel.

As a method of reducing a risk of ischemic stroke onset due to vascular occlusion, a method is conceivable in which the functional device is indwelled in a vein of the brain. This is because veins are on the downstream side of the brain tissue, resulting in a low risk of ischemic stroke or vascular occlusion due to scattering of thrombi. However, since veins have very thin blood vessel walls and greater tortuosity as compared with arteries, there are almost no records of a medical device being indwelled in the veins of the brain and the endovascular treatment using the medical device.

As devices for making the measurement for the brain, the functional device of the present invention can be indwelled in a superficial cerebral venous blood vessel. Here, in order to cause the functional device to access the inside of the brain without damaging the superficial cerebral venous blood vessel and indwell the functional device in the superficial cerebral venous blood vessel, the following two points are essential.

One is a transportation device having an extremely small diameter that enables the functional device to be transported in the superficial cerebral venous blood vessel. The present invention provides this transportation device using a guidewire and a guiding catheter.

The other point is that the functional device should be a flexible functional device having an extremely small diameter that can be indwelled in the superficial cerebral venous blood vessel. The present invention provides this functional device having an extremely small diameter. Description will be provided in the following order.

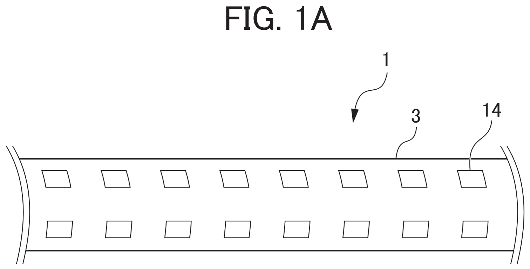

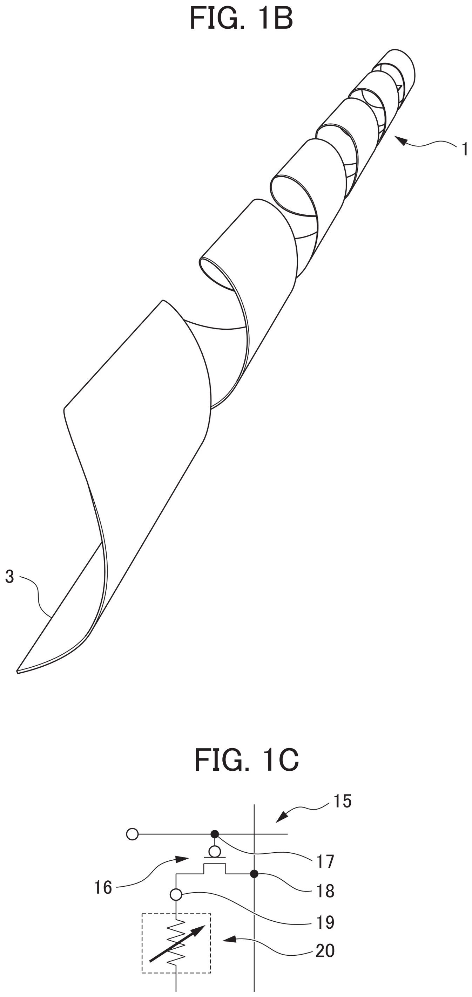

Embodiments of a functional device 1 of the present invention will be described with reference to FIGS. 1A to 4. FIG. 1A is a diagram illustrating the functional device 1 of the present invention. FIG. 1B is a diagram illustrating a form when the functional device 1 is indwelled inside a blood vessel.

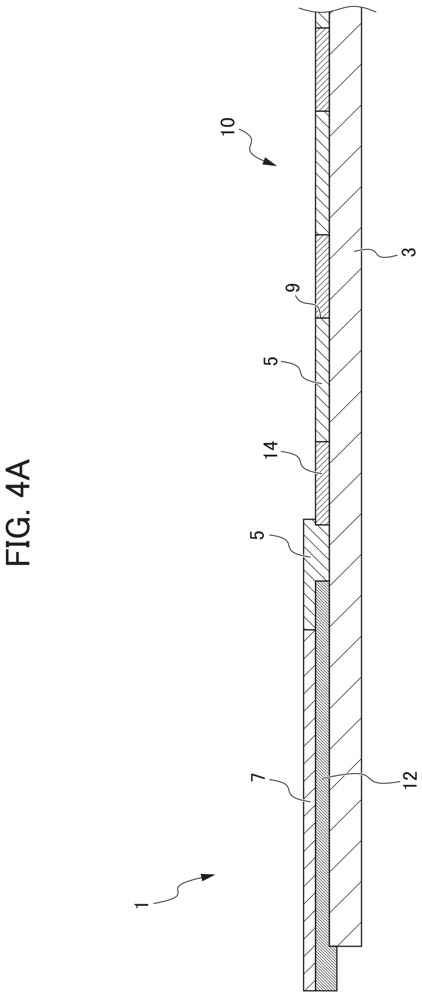

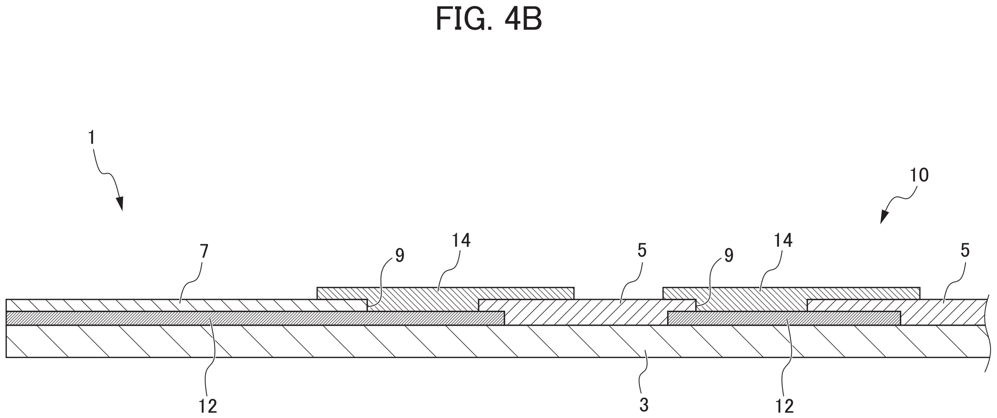

As illustrated in FIG. 1A, the functional device 1 has an elongated rectangular shape in a top view. The short side of the rectangle falls within a range of 0.1 mm or more and 100 mm or less, and the long side of the rectangle falls within a range of 1 mm or more and 2000 or less. It is desirable that a ratio of the long side to the short side of the rectangle falls within a range of 2 or more and 2000 or less. As described later, the functional device 1 is rounded in a helical shape and is disposed in the blood vessel. In a case where the functional device 1 having a rectangular shape in which the ratio of the long side to the short side falls within a range of 2 or more and 2000 or less is rounded in a helical shape and is disposed in the blood vessel, the functional device 1 easily functions at a wide portion in the blood vessel. The functional device 1 includes a substrate 3, and a functional part formed on the substrate 3. The substrate 3 will be described later with reference to FIG. 4.

The functional part has at least one function selected from a sensing part and a stimulation part. The functional device 1 may include a plurality of kinds of functional parts. A case will be described below where the functional part is an electrode 14 functioning as the sensing part. The electrode 14 functions as a sensor for sensing variations in the potential of a living body.

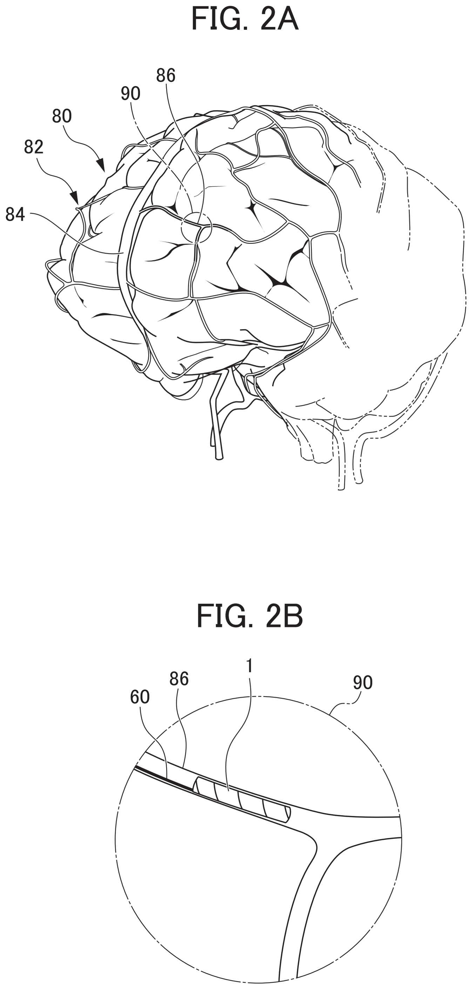

A plurality of electrodes 14 are formed on the substrate 3. The plurality of electrodes 14 can be regularly arranged in a matrix or the like. A connection wire 60 is connected to the electrodes 14. The connection wire 60 is not illustrated in FIG. 1A but is illustrated in FIG. 2B.

The functional device 1 is a sheet-shaped flexible component. Therefore, as illustrated in FIG. 1B, the functional device 1 can be rounded in a helical shape. The functional device 1 can be a spiral electrode. In a state in which the functional device 1 is rounded in a helical shape, the electrodes 14 are located on an outer surface of the substrate 3.

In a state of being rounded in a helical shape, the functional device 1 is indwelled inside the blood vessel. FIG. 2A is a diagram illustrating an overview of a brain 80. FIG. 2B is an enlarged view of a circle 90 in FIG. 2A. As illustrated in FIG. 2A, a plurality of blood vessels 82 are present on a surface of the brain 80. The blood vessels 82 include a venous sinus 84 and a superficial cerebral venous blood vessel 86. The venous sinus 84 is a vein passing through the dura mater, and is a stiff vein. The superficial cerebral venous blood vessel 86 is a vein branching off from the venous sinus 84, and is a soft vein.

As illustrated in FIG. 2B, in a state of being rounded in a helical shape, the functional device 1 is indwelled in the blood vessel. The functional device 1 is indwelled in the fine and soft superficial cerebral venous blood vessel 86. The functional device 1 is thin and flexible. Therefore, the functional device 1 can be indwelled in the fine and soft blood vessel such as the superficial cerebral venous blood vessel 86.

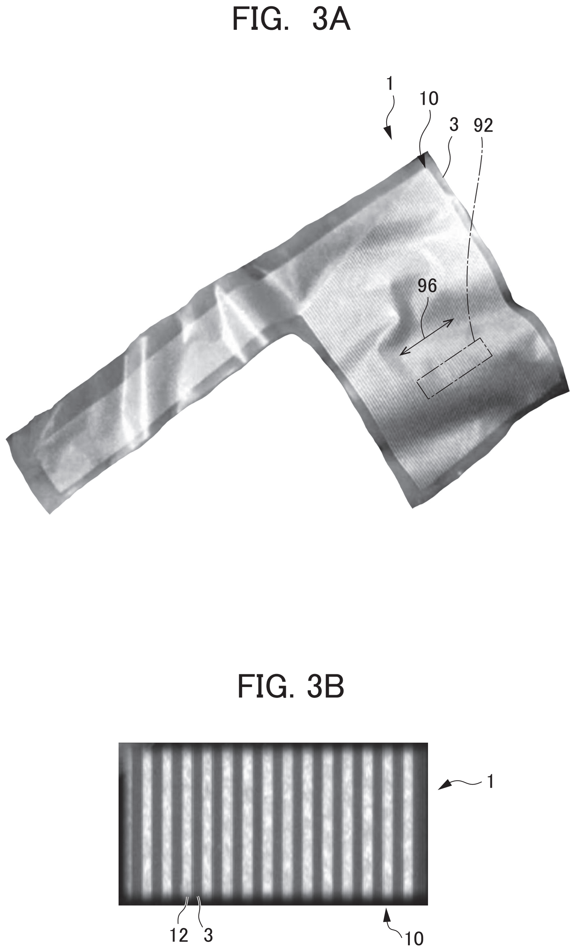

FIG. 3A is a diagram illustrating an example of a functional device 1. As illustrated in FIG. 3A, the functional device 1 includes a substrate 3, and a conductive material arrangement part 10 formed on at least one surface of the substrate 3. The substrate 3 is a portion functioning as a support body in the functional device 1. The conductive material arrangement part 10 is a portion where conductive materials such as an electrode, a wiring, and a circuit are disposed to achieve the electric function. FIG. 3A illustrates a portion where the wirings are formed on the substrate 3. FIG. 3A illustrates a portion of the wirings in a case where the wirings extend from each of a plurality of electrodes arranged on the substrate 3.

The thickness of the substrate 3 can fall within a range of 0.5 µm or more and 50 µm or less. The Young's modulus (the proportional constant between strain and stress calculated from the tension, the compression, and the like, or the stress at 100% strain) of the functional device 1 can be 100 MPa or less.

The rigidity of the substrate 3 is determined by the above-described thickness and Young's modulus of the substrate 3. The degree of self-expansion of the functional device 1 inside the blood vessel 82 is affected by the rigidity of the substrate 3. The degree of self-expansion of the functional device 1 is also affected by the thickness of the substrate 3. The preferable range of the thickness of the substrate 3 is, for example, 0.5 µm or more and 50 µm or less. The self-expansion means that the functional device 1 is deployed by a force of the functional device 1 attempting to deploy by itself when the functional device 1 is discharged into the blood vessel 82.

FIG. 3B is an enlarged view of a square 92 in FIG. 3A. As illustrated in FIG. 3B, a plurality of fine wirings 12 are formed on the substrate 3. For example, in a case where the functional device 1 is configured to be able to detect a 64-channel signal, the wire width of the wirings 12 can be 300 µm or the like. The wire width of the wirings 12 can be 10 µm or less depending on the size of the functional device 1, the circuit configuration, or the like.

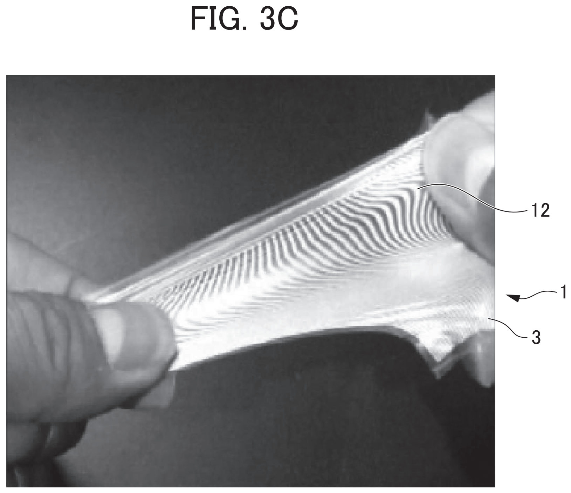

FIG. 3C is a diagram illustrating the state of the functional device 1 when the functional device 1 illustrated in FIG. 3A is elongated in a direction of an arrow 96 in FIG. 3A. As illustrated in FIG. 3C, conductive materials such as the wirings 12 formed on the substrate 3 can follow the elongation or the stretching of the substrate 3. That is, in a case where the conductive material is an electrode, the electrode can follow the elongation or the stretching in the same manner.

A layer configuration and the like of the functional device 1 will be described with reference to FIGS. 4A and 4B. FIGS. 4A and 4B each are a cross-sectional view of the functional device 1. The layer configuration of the functional device 1 illustrated in FIG. 4A and the layer configuration of the functional device 1 illustrated in FIG. 4B are different in the layer on which the electrode 14 is disposed. FIGS. 4A and 4B each illustrate the layer configuration of the functional device 1, but the functional device 1 can adopt the other various layer configurations.

The functional device 1 has flexibility such as extensibility and stretchability as a whole. Therefore, the functional device 1 can be rounded with a small diameter. This enables the functional device 1 to indwell in the fine blood vessel.

As illustrated in FIGS. 4A and 4B, the functional device 1 includes a flexible substrate 3, a wiring 12, an electrode 14, an insulating member 5, and a cover member 7. The functional device 1 has a substantially planar sheet shape as a whole. In particular, in the layer configuration in FIG. 4A, the planarity of the surface is high. However, even in the layer configuration in FIG. 4B, the surface is substantially planar. This is because the thickness of the electrode 14 is thin.

In the functional device 1, a difference between the thickness of a portion where the electrode 14 is disposed and the thickness of a portion where the insulating member 5 is disposed is, for example, 50 µm or less.

The substrate 3 is formed into a sheet shape. The material of the substrate 3 is a flexible resin material (a urethane resin, an acrylic resin, an ester resin, a silicone resin, a modified silicone resin, an imide resin, an epoxy resin, a xylene resin, an ethylene resin, a vinyl resin, cellulose, or the like) including elastomer. The substrate 3 has extensibility and stretchability in an in-plane direction through an external force.

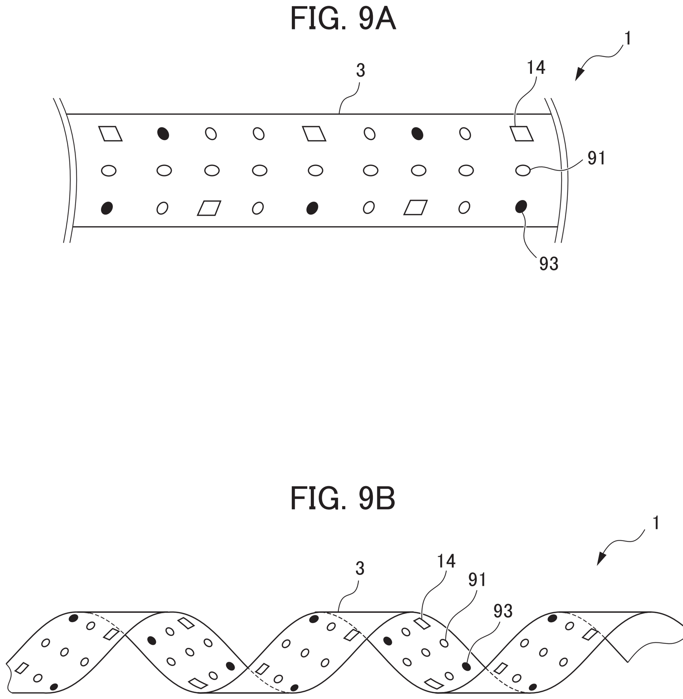

The substrate 3 may include openings that do not obstruct the blood flow. Examples of the openings include through holes 91 passing through the substrate 3. FIG. 9A is a diagram illustrating a modified example of a functional device 1 in which the through holes 91 are formed. FIG. 9B is a diagram illustrating a form when the functional device 1 illustrated in FIG. 9A is indwelled inside the blood vessel 82.

Forming the through holes 91 in the substrate 3 enables red blood cells in blood to pass through the through holes when the functional device 1 is disposed on an inner wall of the blood vessel 82. This enables the red blood cells to flow into the blood vessels branching off from the blood vessel 82 in which the functional device 1 is disposed. The diameter of the through holes 91 is preferably 10 µm or more from the standpoint of allowing the red blood cells to pass through the through holes 91. The diameter of the through holes 91 is more preferably 20 µm or more. However, when the diameter of the through holes 91 becomes too large, the above-described self-expansion may be inhibited. The diameter of the through holes 91 is preferably 1000 µm or less. Furthermore, when the sum of the areas of all the through holes 91 becomes too large, the self-expansion may be inhibited. The sum of the areas of all the through holes 91 is desirably 50% or less of the area of the substrate 3.

The number of the through holes 91 formed in the substrate 3 is not limited to a particular number. The number of the through holes 91 can be determined according to the thickness of the substrate 3.

The arrangement of the through holes 91 is not limited to the particular arrangement. The through holes 91 are preferably arranged such that they are dispersed at substantially constant density. This enables the red blood cells to smoothly flow into the blood vessels branching off from the blood vessel 82. Alternatively, the through holes 91 may be regularly arranged, for example, in a linear manner or in a matrix. Alternatively, as illustrated in FIG. 9A, the through holes 91 may be regularly arranged in combination with the other members such as the electrodes 14. The number and/or arrangement of the through holes 91 is preferably set not to inhibit the functional device 1 from having a helical structure as illustrated in FIG. 9B.

The opening is not limited to a circular hole such as the through hole 91. The opening may be, for example, a polygonal hole, or a linear cut.

The wiring 12 is formed of a conductive material. The conductive material is a bulk-based material in which only a conductor is used or a composite material in which conductors are dispersed in a resin material as a binder. The wiring 12 is arranged on the substrate 3 according to the shape of the electrode or the circuit.

The conductive material has a low Young's modulus. The Young's modulus of the conductive material can be 100 GPa or less or 1 GPa or less. Since the conductive material has a low Young's modulus, the wiring 12 can be elongated or stretched. The wiring 12 can be stretched following the stretching of the substrate 3 in an in-plane direction. The wiring 12 can be formed on the substrate 3 by patterning by means of lithography, a printing method, or the like. The bulk-based material can have structural flexibility to form a horseshoe shape, a honeycomb shape, a mesh shape, or the like. The composite material can show flexibility by its material itself.

The electrode 14 is formed of the same conductive material as the wiring 12. The electrode 14 can be formed into any shape such as a circular shape in a plan view. The position where the electrode 14 is disposed and the number of the electrodes 14 can be arbitrarily set. The wiring 12 is connected to each electrode 14. An amplifier circuit including a transistor may be incorporated between each electrode 14 and the wiring 12. For the transistor, a thin film transistor rich in flexibility, such as an organic field-effect transistor or an organic electrochemical transistor, can be used.

The electrode 14 can be stretched similarly to the wiring 12. The electrode 14 can follow the elongation or the stretching of the substrate 3. The electrodes 14 can be formed on the substrate 3 by patterning by means of lithography, a printing method, or the like. The bulk-based material can have flexibility by structural patterning such as a horseshoe shape, a honeycomb shape, a mesh shape, or the like. The composite material can show flexibility by the mechanical properties of its material itself.

The insulating member 5 is a sheet-shaped cover. The insulating member 5 is disposed on surfaces of the substrate 3, the wiring 12, the electrode 14, and the like. The insulating member 5 is a member for preventing unnecessary electrical conduction between adjacent conductors. The through hole 9 is formed at a position overlapping with the electrode 14 in the insulating member 5. The through hole 9 means a hole formed in the insulating member 5. A portion of the electrode 14 is exposed via the through hole 9. The insulating member 5 is formed of a flexible resin material or the like. The insulating member 5 can follow the elongation or the stretching of the substrate 3.

The cover member 7 is disposed to cover the wiring 12. The cover member 7 prevents the wiring 12 from being unnecessarily electrically connected to the outside. The cover member 7 can be formed of a flexible resin material similarly to the insulating member 5. The cover member 7 can follow the elongation or the stretching of the substrate 3. The insulating member 5 and the cover member 7 can be formed by one member.

The functional device 1 is indwelled in the blood vessel of a living body. Since the functional device 1 is thin and flexible, the functional device 1 can be indwelled in the fine and weak blood vessel such as the superficial cerebral venous blood vessel 86. The functional device 1 is planar, making it difficult to damage the inner wall of the blood vessel.

Since the functional device 1 has the extensibility and the stretchability, the functional device 1 can be brought into close contact with the inner wall of the blood vessel in a state of being curved to conform to the shape of the blood vessel.

In a case where the functional device 1 is indwelled in the superficial cerebral venous blood vessel 86, an electroencephalogram can be measured with high resolution.

The functional device 1 can include a signal processing circuit for amplifying signals, removing noise, and the like, or an electrical circuit for a sensor element, or the like, in addition to the electrodes 14. FIG. 1C is a diagram illustrating an example of an electrical circuit 15 included in the functional device 1. The electrical circuit illustrated in FIG. 1C includes a transistor circuit 16 and a sensor element 20. The transistor circuit 16 includes a gate electrode 17, a source electrode 18, and a drain electrode 19. The sensor element 20 is formed on a side of the drain electrode 19 in the transistor circuit 16. The sensor element 20 functions as a sensor of a type different from the electrode 14 for sensing variations in the potential, and can be, for example, a pressure-sensitive rubber sensor.

The sensor element 20 can be a sensor that detects a pressure, a vibration, a blood flow rate, a current, a voltage, an electric field, a magnetic field, a temperature, light, a chemical quantity, and the like. The chemical quantity includes an oxygen concentration, a glucose metabolism, amyloid β, a NO metabolite, a nitrate ion, a glial cell metabolite, and the like.

The functional device 1 can include a plurality of electrical circuits 15. In a case where the functional device 1 includes a plurality of electrical circuits 15, the electrical circuits 15 can be arranged in a matrix. In this case, the functional device 1 may include gate wires to be connected to a plurality of gate electrodes 17 arranged in the same row or in the same column. In addition, the functional device 1 may include source wires to be connected to a plurality of source electrodes 18 arranged in the same row or in the same column. The plurality of gate wires can be arranged to form a matrix, and the plurality of source wires can be arranged to form a matrix.

In the functional device 1 of the present embodiment, at least a portion of the surface of the functional device 1 is covered with an antithrombotic material. For example, at least any one of at least a portion of the surface of the substrate 3, at least a portion of the surface of the cover member 7, and at least a portion of the surface of the electrode 14 is covered with an antithrombotic material. The whole surface of the functional device 1 may be covered with an antithrombotic material. Examples of the antithrombotic material includes polyvinylpyrrolidone, polyethylene glycol, 2-methacryloyloxyethyl phosphorylcholine, polyacrylic ester (poly(2-methoxyethyl acrylate), poly-(ω-methoxyalkyl acrylate), or the like), an Organic sulfur compound (heparin, heparin derivative, or the like) having anticoagulant activity, a block copolymer (copolymerized with a polyhydroxyalkanoate, an aliphatic polyester, or the like) using a polyalkylene glycol, and a nitrogen-containing polymer having an alkylsulfonic acid group.

In a case where an artifact including an electrode is indwelled inside the blood vessel, thrombi are formed, and the thrombi may cause the clogging inside the blood vessel. In the functional device 1 of the present embodiment, at least a portion of the surface of the functional device 1 is covered with an antithrombotic material. This makes it possible to reduce the formation of thrombi inside the blood vessel without preventing endothelialization even when the functional device 1 is indwelled inside the blood vessel.

The functional device 1 will be described in more detail. The thickness of the substrate 3 falls within a range of 0.5 µm or more and 100 µm or less, preferably 10 µm or less. The thickness of the functional device 1 is preferably 300 µm or less.

The maximum elongation rate of the substrate 3 is preferably 50% or more, more preferably 500% or more. Here, the maximum elongation rate of the substrate 3 means the maximum value in various elongation rates of the substrate 3 in the in-plane direction. The elongation rate of the substrate 3 means a ratio of an amount of elongation in the in-plane direction due to application of a force to the dimension when no force is applied. The elongation rate of 50% means that the dimension when a force is applied is 1.5 times the dimension when no force is applied.

The Young's modulus of the substrate 3 can be 10 GPa or less, preferably 50 MPa or less.

The substrate 3, the insulating member 5, and the cover member 7 can be formed of the same resin material. When these are formed of the same resin material, the functional device 1 can be produced without deteriorating the extensibility and the stretchability of the substrate 3.

The conductors included in the conductive material for the wiring 12, the electrode 14 and the like can include silver, gold, platinum, carbon, copper, aluminum, cobalt, nickel, titanium, iridium, conductive polymer, gel, an ionic liquid, or alloys thereof (composite material). The shape of the conductive material may be the particle shape. The specific shape of the particle shape can be a spherical shape, a needle shape, a flake shape, a nanowire shape, or the like. The particle aspect ratio falls within a range of preferably 1 or more and 1000 or less, more preferably 1 or more and 500 or less. Here, the aspect ratio means the ratio between the longest dimension and the shortest dimension of a three-dimensional body.

The electrode 14 may be subjected to a surface treatment. Examples of the surface treatment include an oxygen plasma treatment, a corona discharge treatment, and coating with an antithrombotic material. When the electrode 14 is subjected to the surface treatment, the adhesion between the inner wall of the blood vessel 82 and the electrode 14 can be improved. When the adhesion between the inner wall of the blood vessel 82 and the electrode 14 is improved, the contact impedance between the inner wall of the blood vessel 82 and the electrode 14 decreases, making it possible to measure a weak electroencephalogram. In addition, the effect of preventing the formation of thrombi can be obtained. The preferable range of the contact impedance between the inner wall of the blood vessel 82 and the electrode 14 is 10 kΩ or less.

A resin binder used for the conductive material for the wiring 12, the electrode 14, and the like can include a flexible resin material containing elastomer such as a urethane resin, an acryl resin, an ester resin, a silicone resin, a modified silicone resin, an imide resin, an epoxy resin, a xylene resin, an ethylene resin, a vinyl resin, and cellulose.

The Young's modulus of the resin binder is preferably equivalent to the Young's modulus of the substrate 3 or smaller than the Young's modulus of the substrate 3. The Young's modulus of the resin binder is preferably 50 MPa or less. The resin binder may include only one kind of flexible resin material or a plurality of kinds of flexible resin materials.

The conductive material for the wiring 12, the electrode 14, and the circuit can be formed on the substrate 3 by means of a printing method. The specific examples of the printing method include a screen printing method, an inkjet printing method, a gravure printing method, an offset printing method, a dispenser printing method, and a mask printing method. Of these printing methods, the screen printing method is preferable. This is because the screen printing method is excellent in fine resolution and thick film stability.

In a case where the conductive material for the wiring 12, the electrode 14, and the like is formed by the printing method, first, the above-described conductive paste including the conductive particles, the resin binder, and an organic solvent is prepared. Then, the prepared conductive paste is printed on the surface of the substrate 3 or the like.

After being printed and dried, the conductive paste has extensibility and stretchability. The wiring 12, the electrode 14, the circuit, and the like preferably have the elongation rate of 50% or more.

The thicknesses of the wiring 12, the electrode 14, and the like are preferably 50 µm or less, more preferably 15 µm or less.

The functional device 1 functions as an electroencephalogram measurement device having an extremely small diameter by being indwelled inside the blood vessel.

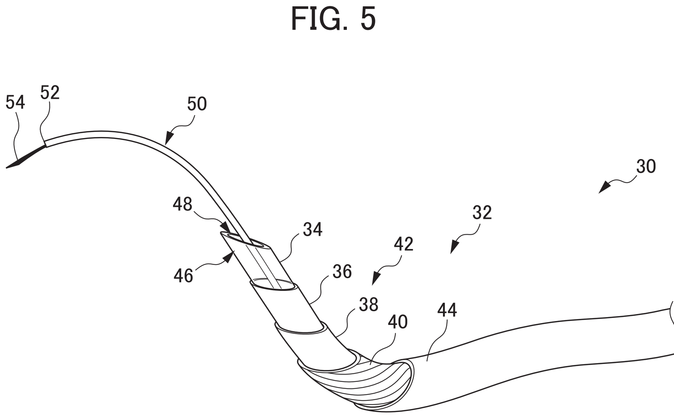

A method of indwelling the functional device 1 inside the blood vessel will be described. The functional device 1 is indwelled inside the blood vessel using a transportation device. A transportation device 30 will be described with reference to FIG. 5. FIG. 5 is a diagram illustrating the transportation device 30. As illustrated in FIG. 5, the transportation device 30 includes a guiding catheter 32 and a guidewire 50.

The guiding catheter 32 includes a catheter 34, an electrode 36, a pressure-sensitive electroconductive material 38, a readout circuit 40, and an antithrombotic protective film 44.

The catheter 34 is a hollow tube. The catheter 34 has a cavity 48 therein.

The electrode 36 is disposed to cover the periphery of the catheter 34. The electrode 36 can be a gold electrode or the like.

The pressure-sensitive electroconductive material 38 is disposed to cover the electrode 36. The readout circuit 40 is disposed to cover the pressure-sensitive electroconductive material 38.

The electrode 36, the pressure-sensitive electroconductive material 38, and the readout circuit 40 constitute a multi-layered thin-film sensor array 42. The multi-layered thin-film sensor array 42 can function as a biological sensor that detects a pressure, a blood flow rate, an electric field, a magnetic field, a temperature, a chemical quantity, and the like. The chemical quantity includes an oxygen concentration, a glucose metabolism, amyloid β, a NO metabolite, a nitrate ion, a glial cell metabolite, and the like.

The antithrombotic protective film 44 is disposed to cover the readout circuit 40. The antithrombotic protective film 44 reduces the formation of thrombi inside the blood vessel.

The guidewire 50 is disposed inside the catheter 34. The guidewire 50 is disposed in the cavity 48 of the catheter 34.

The guidewire 50 can freely change the direction of its distal end 52. An actuator 54 may be provided at the distal end 52 of the guidewire 50. Providing the actuator 54 at the distal end 52 enables the guidewire 50 to more freely change the direction of its distal end 52.

The same sensor as the above-described multi-layered thin-film sensor array 42 may be disposed on the surface near the distal end of the guidewire 50. Another kind of sensor such as a piezoelectric sensor may be disposed on the surface near the distal end of the guidewire 50. Alternatively, the same sensor and another kind of sensor may be disposed on the surface near the distal end of the guidewire 50. The same antithrombotic protective film as the above-described antithrombotic protective film 44 may be disposed on the outermost surface of the guidewire 50.

The method of indwelling the functional device 1 inside the blood vessel includes two steps. A first step is a guiding catheter disposing step. A second step is a functional device indwelling step.

The guiding catheter disposing step is a step in which a first end portion 46 of the guiding catheter 32 is disposed at a predetermined position inside the blood vessel.

The functional device indwelling step is a step in which after the first end portion 46 of the guiding catheter 32 is disposed at the predetermined position inside the blood vessel, the functional device 1 is discharged from the first end portion 46 of the guiding catheter 32 into the blood vessel so that the functional device 1 is indwelled inside the blood vessel. Description will be provided in the following order.

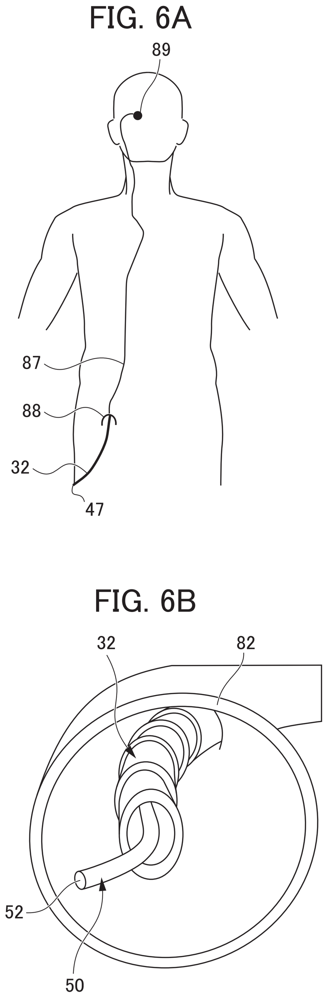

Note that the guiding catheter 32 can be inserted into the body from the jugular vein, the femoral vein, or the like. FIG. 6A is a diagram illustrating a state in which the guiding catheter 32 is inserted into the body from outside the body. FIG. 6A illustrates a state in which the guiding catheter 32 is inserted into the femoral vein 87 via a catheter insertion port 88.

After the guiding catheter 32 is inserted into the body, the functional device 1 is indwelled in the brain in the following order so that an electrocorticogram (ECoG) 89 can be measured. In addition to the electrocorticogram, a local field potential (LFP) signal in the brain can be measured.

FIG. 6B is a diagram illustrating the guiding catheter 32 and the guidewire 50 inside the blood vessel 82. As illustrated in FIG. 6B, in the guiding catheter disposing step, the guiding catheter 32 is guided by the guidewire 50, and is disposed at the predetermined position inside the blood vessel. The distal end 52 of the guidewire 50 can freely change its direction. As illustrated in FIG. 2A, the blood vessels 82 are tortuous in the brain 80. In order to indwell the functional device 1 in the superficial cerebral venous blood vessel 86, it is necessary to dispose the first end portion 46 of the guiding catheter 32 at the predetermined position through fine branches in the blood vessels 82. At this time, the guidewire 50 traverses the inside of the blood vessels 82 prior to the first end portion 46 of the guiding catheter 32. Then, the guiding catheter 32 is guided by the guidewire 50, and traverses the inside of the blood vessels 82. This enables the first end portion 46 of the guiding catheter 32 to traverse to reach the predetermined position even when the continuous branches are present in the blood vessels 82, even when the blood vessel 82 branches off in multiple directions, or even when it is necessary to change the path at an acute angle at the branch.

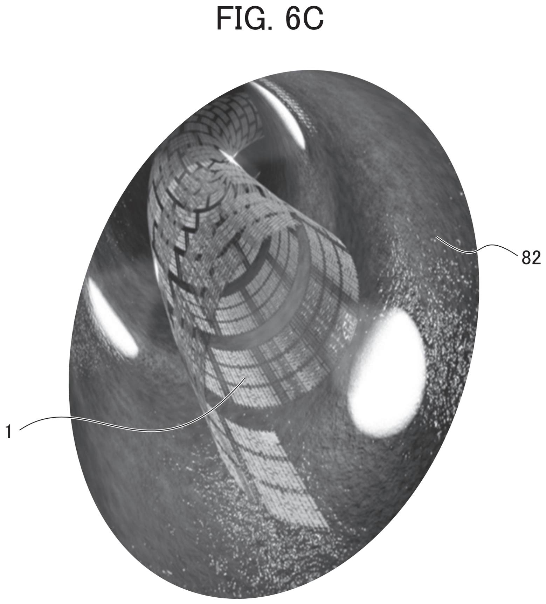

FIG. 6C is a diagram illustrating the functional device 1 inside the blood vessel 82. After the first end portion 46 of the guiding catheter 32 is disposed at the predetermined position, the functional device 1 is discharged from the first end portion 46 of the catheter 34 into the blood vessel 82. The functional device 1 is located in the vicinity of the first end portion 46 in the cavity 48 of the catheter 34 before being discharged into the blood vessel 82. When being discharged from the cavity 48 of the catheter 34 into the blood vessel 82, the functional device 1 is deployed into a helical shape inside the blood vessel 82, as illustrated in FIG. 6C. This is because a force of the functional device 1 attempting to deploy by itself is exerted on the functional device 1 when the functional device 1 is discharged into the blood vessel 82. The deployed functional device 1 contacts the inner wall of the blood vessel 82. Then, the functional device 1 is indwelled at the predetermined position inside the blood vessel 82.

The above-described deployment by a force of the functional device 1 attempting to deploy by itself is also referred to as self-expand or self-expansion. In order to pressure-bond the self-expanded functional device 1 to the inner wall of the blood vessel 82 at an appropriate pressure, the thickness, the Young's modulus, the rigidity, and the size of the substrate 3 as described above are preferably set within appropriate ranges.

In a state in which the functional device 1 is indwelled inside the blood vessel 82, the electrodes of the functional device 1 can face the inner wall of the blood vessel 82. The functional device 1 is in planar contact with the inner wall of the blood vessel 82. Therefore, the stress applied to the blood vessel 82 from the functional device 1 is dispersed, making it possible to reduce burden on the blood vessel 82. The planar functional device 1 can be brought in close contact with the blood vessel, making it possible to induce the endothelialization.

The procedure for discharging the functional device 1 from the cavity 48 of the catheter 34 into the blood vessel 82 is not limited to particular procedure. In a state in which the first end portion 46 of the guiding catheter 32 is disposed at the predetermined position, the functional device 1 can be inserted into the cavity 48. Alternatively, in a state in which the functional device 1 is disposed inside the cavity 48 in advance, the first end portion 46 of the guiding catheter 32 may be disposed at the predetermined position inside the blood vessel 82. Description will be provided in the following order.

Description will be provided about the procedure for inserting the functional device 1 into the guiding catheter 32 after the guiding catheter 32 is disposed inside the blood vessel 82. Of the two end portions of the guiding catheter 32, the other end portion which is not the first end portion 46 is referred to as a second end portion 47. The second end portion 47 is illustrated in FIG. 6A.

In the functional device indwelling step, the functional device 1 is inserted into the cavity 48 inside the guiding catheter 32 from the second end portion 47 of the guiding catheter 32. The functional device 1 is disposed inside the cavity 48 in a state of being rounded in a helical shape. Then, the functional device 1 is pushed by a piston-shaped member (not illustrated) to move the functional device 1 inside the cavity 48. The functional device 1 is moved to the first end portion 46 in the cavity 48. Thereafter, the functional device 1 is discharged from the first end portion 46 into the blood vessel 82.

In order to dispose the functional device 1 at the appropriate position within the body, the position of the functional device 1 within the body can be preferably grasped by means of X-rays. In order to grasp the position of the functional device 1 by means of X-rays, a portion that does not transmit X-rays is preferably formed in the functional device 1. The portion that does not transmit X-rays is referred to as a marker 93 (X-ray marker). The functional device 1 provided with the markers 93 is illustrated in FIGS. 9A and 9B.

The marker 93 can be formed of, for example, a material that does not transmit X-rays, such as gold and platinum, that is, a material that blocks X-rays. The marker 93 can be also formed of an alloy of platinum and iridium, tantalum, or the like. The size of the marker 93 provided in the functional device 1 can be, for example, 10 µm or more in diameter. The thickness of the marker 93 can be, for example, 10 µm or more from the standpoint of blocking X-rays. When the thickness of the marker 93 is 10 µm or less, the flexibility of the functional device 1 can be ensured. The markers 93 can be provided, for example, at positions in front and rear of the functional device 1, positions on both sides of the functional device 1 in the width direction, and the like. Here, regarding the front and rear and the width direction, the insertion direction into the blood vessel 82 is defined as the front, and a direction intersecting the front-rear direction is defined as the width direction. Alternatively, the markers 93 may be regularly disposed in combination with the other members such as the electrodes 14 as illustrated in FIGS. 9A and 9B.

The markers 93 are not connected to the wirings 12 and the connection wire 60, and therefore, need not function electrically. In a case where the electrode 14 is formed of the material that does not transmit X-rays, such as platinum, the electrode 14 may also serve as the marker.

In a case where the first end portion 46 of the guiding catheter 32 is disposed at the predetermined position in a state in which the functional device 1 is disposed inside the cavity 48, before the guiding catheter 32 is inserted into the body, the functional device 1 is inserted into the guiding catheter 32 and the functional device 1 is moved to the first end portion 46 by the piston-shaped member or the like, as described above.

The connection wire 60 is connected to the functional device 1 indwelled inside the blood vessel 82 as illustrated in FIG. 2B. The connection wire 60 is a wiring that connects with an apparatus outside the blood vessels or the like, which will be described later.

In the above description, the guiding catheter 32 includes the multi-layered thin-film sensor array 42. Note that the guiding catheter 32 may include only the catheter 34. The transportation device 30 includes the catheter 34 and the guidewire 50, making it possible to function as the transportation device 30.

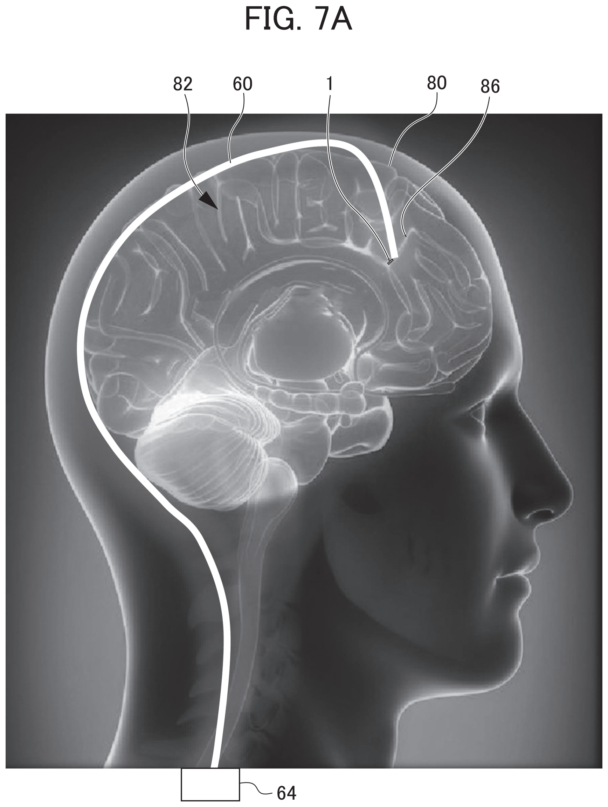

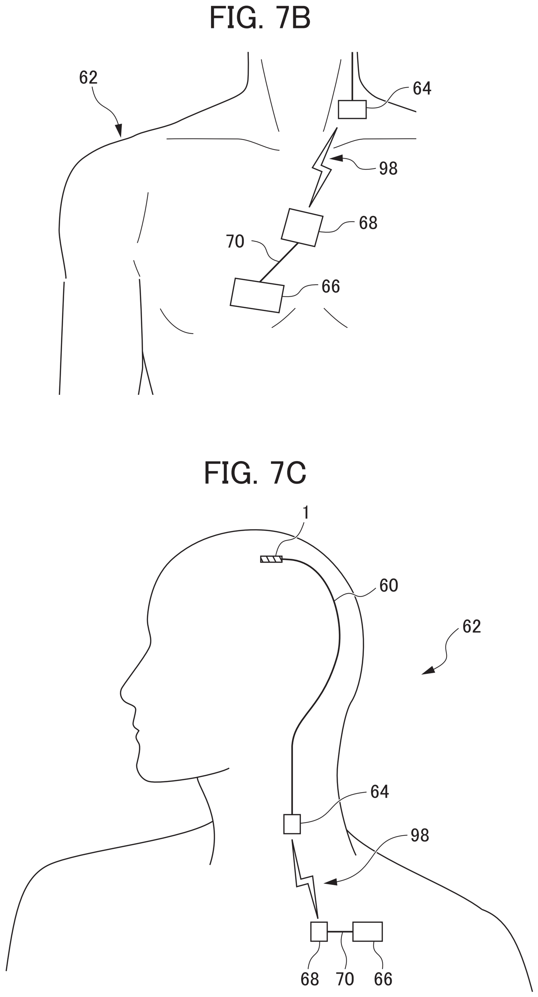

The entire extremely small diameter-intravascular measuring system including the functional device 1 and the apparatus disposed outside the blood vessels is referred to as a biological interface system 62. The biological interface system 62 will be described with reference to FIGS. 7A to 7C. FIGS. 7A and 7B each are a diagram illustrating a portion of the biological interface system 62. FIG. 7C is a diagram illustrating the entire biological interface system 62. FIG. 7A illustrates a signal transmission path from the functional device 1 to a relay 64. FIG. 7B illustrates an external receiving apparatus 66 disposed outside the body and an antenna 68. In FIGS. 7B and 7C, an arrow 98 indicates wireless connection.

The biological interface system 62 is a system that transmits, to an analysis device and the like, an electroencephalogram and the like measured by the functional device 1. The biological interface system 62 includes the relay 64, the external receiving apparatus 66, and the antenna 68. The relay 64 is disposed outside the blood vessels 82. The external receiving apparatus 66 and the antenna 68 are disposed outside the body.

Note that the transmission at high frequency or the like using the external receiving apparatus 66 and the antenna 68, which will be described below, is illustrative of transmission by wireless. Other examples of transmission by wireless include infrared communication. In a case of infrared communication, the relay 64 inside the body is formed by a light emitting element using a light emitting diode. The antenna 68 and external receiving apparatus 66 outside the body can be formed by light receiving elements using photodiodes. As a method of modulating an analog signal in the infrared communication, for example, pulse width modulation (PWM) can be used.

Examples of advantages in using the infrared communication are described below. Infrared light has a superior in-body passing characteristic. The infrared communication can be applied to both of a low-frequency analog signal and an AD data stream. Pinpoint communication is possible using a transmission and reception device. That is, the antenna requiring the area becomes unnecessary, making it possible to reduce the arrangement area. The communication does not receive electromagnetic interference from the surroundings. The radiation signal strength is not regulated by the radio law. The driving circuit and the reception circuit can be simplified.

As illustrated in FIG. 7A, the relay 64 is connected to the functional device 1 disposed in the superficial cerebral venous blood vessel 86 via the connection wire 60. The relay 64 is an apparatus that is first connected to the functional device 1. The relay 64 has at least one function selected from functions of amplifying a signal from the functional device 1 and removing noise from the signal from the functional device 1, wirelessly transmitting the signal to an external receiver set outside the body, and the like.

The relay 64 can be disposed subcutaneously in the neck or the like. Amplifying the signal and removing noise from the signal are preferably performed near a transmission source of the signal. The connection wire 60 is drawn out to the outside of the blood vessel 82 in the jugular vein, and is connected to the relay 64 disposed subcutaneously in the neck. This makes it possible to efficiently amplify the signal and remove the noise from the signal. Alternatively, the relay 64 can be also disposed on the position other than the neck.

The external receiving apparatus 66 is wirelessly connected to the relay 64. The external receiving apparatus 66 is disposed on the chest or the like, as illustrated in FIG. 7B. The relay 64 and the external receiving apparatus 66 can be connected to each other via a subcutaneous wiring. The external receiving apparatus 66 has at least one function selected from functions of a wireless communication circuit (including registered trademark) such as a central processing unit (CPU), an analog-to-digital converter (ADC), and bluetooth low energy (BLE), the other signal processing, and power control. The external receiving apparatus 66 can have a function of a power supply in addition to the above-described signal processing and communication processing. A battery may be provided in the external receiving apparatus 66.

The antenna 68 is connected to the external receiving apparatus 66 via a wiring 70, as illustrated in FIGS. 7B. The antenna 68 is disposed on the chest or the like. The antenna is preferably disposed in the vicinity of the external receiving apparatus 66. The wiring 70 can be disposed outside the body. The antenna 68 receives the signal and the like measured by the functional device 1 and transmitted to the analysis device and the like. The antenna 68 can also perform wireless power transmission to the relay 64.

For the above-described wireless power transmission, electromagnetic inductive power supply and magnetic-field resonance power supply can be used, for example. The above-described battery may be also provided in the relay 64 inside the body. The battery provided in the relay 64 can be, for example, a button battery or a chip battery.

FIG. 7C illustrates an arrangement example of the entire biological interface system 62. The functional device 1 is disposed in the brain, the relay 64 is disposed in the neck, and the external receiving apparatus 66 and the antenna 68 are disposed on the chest. The functional device 1 and the connection wire 60 are disposed inside the blood vessel. The relay 64 and the external receiving apparatus 66 are connected to each other via the antenna 68 by radio waves or the like. The external receiving apparatus 66 and the antenna 68 are disposed outside the body.

The electrocorticogram (ECoG), the local field potential (LFP) signal in the brain, particularly in the deep brain, and the like that are measured by the functional device 1 can be transmitted to the analysis device and the like by the above-described biological interface system 62.

Note that the above-described configuration of the biological interface system 62, apparatuses included in the biological interface system 62, arrangement of the apparatuses, functions of the apparatuses, method of connecting the apparatuses, and the like are examples. The configuration and the like of the biological interface system 62 can be appropriately changed. A ground potential in the circuit can be set to the potential on a human body such as ears.

The biological interface system 62 may receive the signal from the external apparatus in addition to transmitting the signal to the analysis device and the like. When the functional device 1 applies stimulation to tissue of a living body, the biological interface system 62 may receive a control signal from the external apparatus.

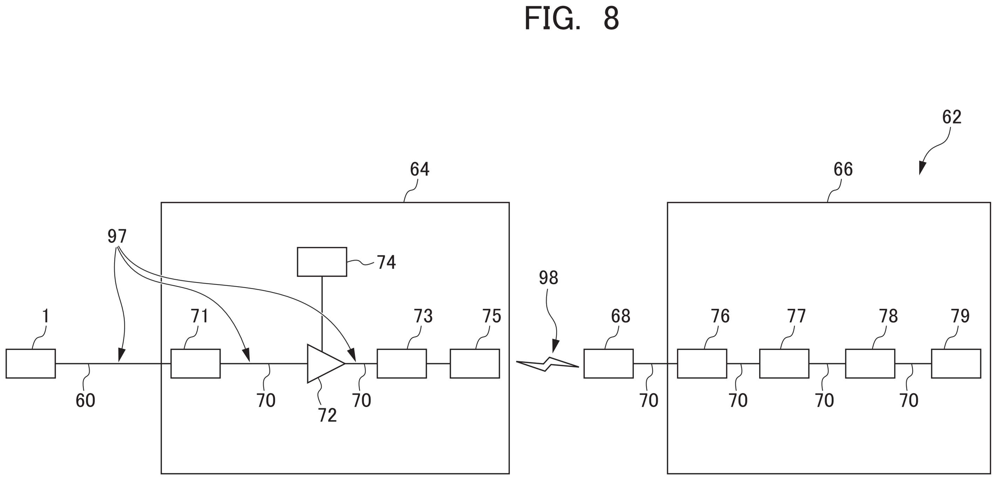

Examples of circuits and the like included in the relay 64 and the external receiving apparatus 66 will be described with reference to FIG. 8. FIG. 8 is a block diagram illustrating a circuit arrangement in the biological interface system 62. As illustrated in FIG. 8, the relay 64 includes a filter 71, a low-noise amplifier 72, a multiplexer 73, and a radio transmitter 75. These are sequentially connected in series. A power supply 74 such as a battery is connected to the low-noise amplifier 72.

A plurality of filters 71 and a plurality of low-noise amplifiers 72 may be included. The filter 71 can include a plurality of low pass filters (LPFs) or a plurality of band pass filters (BPFs). In a case where an electroencephalogram is acquired by the functional device 1, the cut-off frequency of the LPF is normally 50 Hz or less, and the band of BPF falls within a range of 0.5 Hz or more and 50 Hz or less. As the low-noise amplifier 72, for example, an amplifier with noise of 5nV/VHzQ@f = 10 Hz or less can be used.

The numbers of connection wires 60 and wirings 70 indicated by arrows 97 can be plural corresponding to the number of channels of the functional device 1. Here, the number is defined as n.

The external receiving apparatus 66 performs various kinds of signal processing. The external receiving apparatus 66 includes a radio receiver 76, an analog-to-digital converter 77, a signal processing circuit 78, and a data transmission circuit 79. These are sequentially connected in series. The radio receiver 76 can use a high-frequency radio receiver using a frequency band of any of 100 MHz to 5 GHz, for example. Alternatively, the radio receiver 76 can utilize not only high-frequency radio but also optical communication using infrared light or the like
The antenna 68 is connected to the radio receiver 76. The signal subjected to various kinds of processing in the signal processing circuit 78 is connected to a control apparatus such as PC through the data transmission circuit 79. Note that the data signal may be wiredly or wirelessly transmitted to the PC.

The wireless connection indicated by an arrow 98 does not need to use a plurality of (n) radio waves. This is because a plurality of signals are multiplexed on one signal line by the multiplexer 73.

The characteristics and effects of the present invention will be described below. The blood vessel is very soft tissue. Therefore, there is a risk that the blood vessel is perforated by insertion of an artifact. In the present invention, the measurement device and its transporter are produced from a soft functional organic material such as gel. This makes it possible to reduce a risk that the tissue such as a blood vessel in a living body is damaged.

In a case where an artifact is indwelled inside the blood vessel, thrombi are formed inside the blood vessel, and there is a risk that the thrombi cause the clogging inside the blood vessel. In the present invention, the surface of the member to be inserted into the blood vessel is covered by an antithrombotic material. This can prevent the formation of thrombi inside the blood vessel.

According to the present invention, various medical apparatuses can be used inside the blood vessel. The advanced medical treatment can be provided without imposing large invasiveness on a living body.

According to the present invention, the sophistication of the treatment of the inside of the blood vessel enables the surgery with less invasiveness. Furthermore, it is possible to respond to the medical technology needs for measurement in the brain with low invasiveness and with high precision resulting from the rapid increase in the number of patients with brain-associated disease such as a dementia in the world. The methods of using the functional device 1 are diverse. For example, the functional device 1 can be disposed in the superficial cerebral venous blood vessel of a person for use only during surgery, and after the surgery, can be removed. The functional device 1 can be disposed in the superficial cerebral venous blood vessel of a person to continue acquiring biosignals such as an electroencephalogram in the daily life of the person.

As described above, the extremely small diameter-intravascular measuring system according to the present invention enables access to a broad brain surface from the superficial cerebral venous blood vessel with extremely low invasiveness. The extremely small diameter-intravascular measuring system includes an extremely small diameter biometric and stimulation device and a transportation device. The extremely small diameter-intravascular measuring system achieves indwelling of the functional device not inside the venous sinus which is a stiff vein passing through the dura mater but inside a soft superficial cerebral venous blood vessel.

The functional device having an extremely small diameter is indwelled inside the superficial cerebral venous blood vessel. It enables measurement of the brain activity over a long term from the superficial cerebral venous blood vessel via the flexible helical electrode.

The transportation device transports the functional device having an extremely small diameter to the superficial cerebral venous blood vessel and indwells the functional device there. The transportation device enables the measurement with extremely low invasiveness from the superficial cerebral venous blood vessel and the electrical stimulation with extremely low invasiveness to the superficial cerebral venous blood vessel via the guidewire in which the electrode is disposed.

The cerebral blood vessel is extremely fine, and its shape is complex. In particular, the vein has a complex shape. Therefore, the difficulty in accessing the inside of the cerebral blood vessel is high. Indwelling a foreign substance inside the blood vessel involves a risk of vascular occlusion due to formation of thrombi.

In the present invention, the safety and operability inside the blood vessels can be achieved by the ultra-thin film sensor and the guidewire functioning as an actuator. Furthermore, the antithrombotic material makes it possible to reduce a risk of vascular occlusion due to indwelling of the ultra thin-film electrode and the sensor inside the cerebral venous blood vessel.

The ultra-flexible helical electrode utilizing polymer technology enables indwelling of the electrode in a vein that is not suitable for indwelling of a stent because of weak blood vessel.

The guidewire mounted with the sensor enables measurement of ECoG and LFP in the blood vessels in a short period of time such as during surgery. Note that the sensor may cover only the distal end of the guidewire or may cover the entire guidewire.

The functional device having an extremely small diameter may have a plurality of channels such as 16 channels.

Each of the guidewire and the guiding catheter may be mounted with a sensor. The sensor-mounted guidewire and the sensor-mounted guiding catheter are operated in conjunction with each other and are complementarily used, making it possible to safely transport the device inside the blood vessels. In addition, it enables more complex surgical technique.

The sensor-mounted guidewire or the sensor-mounted guiding catheter enables the real-time measurement of a displacement amount, a speed, a direction, a stress, a blood flow rate, and the like, the multimodal measurement by co-mounted sensors of various kinds, the control of neural activity by the electrical stimulation, and the like.

Furthermore, the sensor-mounted guidewire enables access to narrow blood vessels by utilizing its small diameter. The sensor-mounted guiding catheter enables transportation of the device and medication by utilizing its lumen.

The embodiments of the present invention have been described above; however, the present invention is not limited to the aforementioned embodiments, and various modifications, alterations, and combinations are possible.

The functional device 1 of the present invention is configured to measure variations in the potential via the electrodes 14, but the variations in the potential to be measured are not limited to the variations in the potential derived from an electroencephalogram. A place in which the functional device 1 is indwelled is not limited to the superficial cerebral venous blood vessel. A place in which the functional device 1 is indwelled may be a cerebral venous blood vessel or cerebral arterial blood vessel other than the superficial cerebral venous blood vessel, or a blood vessel of a part other than the brain. A place in which the functional device 1 is indwelled may be tissue other than blood vessels, such as an intraspinal space, a cerebral ventricle, a cerebral aqueduct, a cerebral cistern, and visceral organs. The functional device 1 can be used as a device that measures various variations in potential inside the body.

The functional device 1 can be used for various applications and devices that require the measurement of variations in potential inside the body. For example, the electroencephalogram measured via the functional device 1 is subjected to signal processing, so that the various states of the living body can be determined. Specifically, the frequency components included in the electroencephalogram are analyzed, so that a sleep state and a mental state can be analyzed.

The functional device 1 can be used for the BMI device. In the BMI device, the electroencephalogram is used as a control signal for manipulating prosthetic hands. When the functional device 1 is used for the BMI device, the BMI with extremely low invasiveness can be achieved.

The biosignal measured by the functional device 1 may be a signal derived from variations in potential, for example, is not limited to the electroencephalogram or electrocardiogram, and may be a signal derived from mechanical vibration or a signal derived from the light such as a blood oxygen level. For example, the sensor part as a functional part is not limited to the electrode, and may be a vibration detection part (piezoelectric element or the like) that detects the mechanical vibration or may be a light detection part (photodiode or the like) that detects the light.

The functional device 1 may also be not only a device that measures biosignals but also a device having a stimulation part that applies stimulation to the living body. For example, the simulation part may be an electrode for applying the electrical stimulation. The stimulation applied by the stimulation part of the functional device 1 may be the stimulation of the mechanical vibration by the piezoelectric element or the like, for example, ultrasonic vibration, or the light stimulation by the photodiode or the like, in addition to the electrical stimulation. The functional device 1 may be a device that measure biosignals and applies the stimulation to a living body. The functional device 1 may be a device that has the sensor part and the stimulation part. The shape of the functional device 1 need not be a rectangular shape, and may be a square, an elliptical shape, or the like. The functional device 1 preferably has a uniform thickness, but may have a hole in a portion thereof. For example, the functional device 1 need not be disposed in a helical shape in the blood vessel. It is only required that the functional device 1 is disposed to function in the blood vessel.
(1) A functional device that is indwelled inside a blood vessel of a living body, and
   performs at least one selected from measuring activity of tissue outside the blood vessel and applying stimulation to the tissue, the functional device comprising:
   a sheet-shaped flexible substrate;
   at least one functional part formed on the substrate; and
   a wiring formed on the substrate and connected to the functional part, wherein
   at least any one of at least a portion of a surface of the substrate and at least a portion of a surface of the functional part is covered with an antithrombotic material.
(2) The functional device described in (1), wherein
   the functional part has at least one function selected from an electrode and a sensor.
(3) The functional device described in (1) or (2), wherein the sensor detects at least one selected from a vibration, a pressure, a current, a voltage, and light.
(4) The functional device described in any one of (1) to (3), wherein
   a thickness of the substrate falls within a range of 0.5 µm or more and 50 µm or less.
(5) The functional device described in any one of (1) to (4), wherein
   a Young's modulus falls within a range of 1 kPa or more and 10 GPa or less.
(6) The functional device described in any one of (1) to (5), wherein
   the blood vessel is a superficial cerebral venous blood vessel, and
   the tissue is nervous tissue.
(7) A guiding catheter that has a cavity inside the guiding catheter and is flexible, wherein
   the functional device described in any one of (1) to (6) is disposed in the cavity.
(8) The guiding catheter described in (7), wherein
   the functional device is disposed in the cavity in a state of being rounded in a helical shape.
(9) A biological interface system comprising:
   a relay that is connected to the functional device described in any one of (1) to (6) and is disposed outside the blood vessel;
   an external receiving apparatus that is wirelessly connected to the relay and is disposed outside a body; and
   an antenna that is connected to the external receiving apparatus and is disposed outside the body.
(10) The biological interface system described in (9), wherein
   at least one selected from the relay and the external receiving apparatus performs at least one selected from amplifying a signal from the functional device and removing noise from the signal.
(11) A method of indwelling a functional device, the method comprising:
   a guiding catheter disposing step in which a first end portion of a guiding catheter is disposed at a predetermined position inside a blood vessel; and
   a functional device indwelling step in which at the first end portion, a sheet-shaped flexible functional device is discharged from an inside of the guiding catheter into the blood vessel so that the functional device is indwelled inside the blood vessel.
(12) The method of indwelling a functional device described in (11), wherein
   the guiding catheter includes a guidewire, and
   in the guiding catheter disposing step, the guiding catheter is guided by the guidewire and is disposed at a predetermined position inside the blood vessel.
(13) The method of indwelling a functional device described in (11) or (12), wherein
   when two end portions of the guiding catheter are defined as the first end portion and a second end portion,
   in the functional device indwelling step,
   the functional device is inserted into a cavity inside the guiding catheter from the second end portion of the guiding catheter, and
   after the functional device is moved to the first end portion of the guiding catheter, the functional device is discharged into the blood vessel.
(14) The method of indwelling a functional device described in (11) or (12), wherein
   in the guiding catheter disposing step,
   in a state in which the functional device is disposed at the first end portion in a cavity inside the guiding catheter in advance, the first end portion of the guiding catheter is disposed at a predetermined position inside the blood vessel.

### EXPLANATION OF REFERENCE NUMERALS

- 1: Functional device
- 3: Substrate
- 5: Insulating member
- 7: Cover member
- 9: Through hole
- 10: Conductive material arrangement part
- 12: Wiring
- 14: Electrode
- 16: Transistor circuit
- 17: Gate electrode
- 18: Source electrode
- 19: Drain electrode
- 20: Sensor element
- 30: Transportation device
- 32: Guiding catheter
- 34: Catheter
- 36: Electrode
- 38: Pressure-sensitive electroconductive material
- 40: Readout circuit
- 42: Multi-layered thin-film sensor array
- 44: Antithrombotic protective film
- 46: First end portion
- 47: Second end portion
- 48: Cavity
- 50: Guidewire
- 52: Distal end
- 54: Actuator
- 60: Connection wire
- 62: Biological interface system
- 64: Relay
- 66: External receiving apparatus
- 68: Antenna
- 70: Wiring
- 71: Filter
- 72: Low-noise amplifier
- 73: Multiplexer
- 74: Power supply
- 75: Radio transmitter
- 76: Radio receiver
- 77: Analog-to-digital converter
- 78: Signal processing circuit
- 79: Data transmission circuit
- 80: Brain
- 82: Blood vessel
- 84: Venous sinus
- 86: Superficial cerebral venous blood vessel
- 87: Femoral vein
- 88: Catheter insertion port
- 89: Electrocorticogram
- 91: Through hole
- 93: Marker

## Claims

1. A functional device that is indwelled inside a blood vessel of a living body, and
performs at least one selected from measuring activity of tissue outside the blood vessel and applying stimulation to the tissue, the functional device comprising:
a sheet-shaped flexible substrate;
at least one functional part formed on the substrate; and
a wiring formed on the substrate and connected to the functional part, wherein
at least any one of at least a portion of a surface of the substrate and at least a portion of a surface of the functional part is covered with an antithrombotic material.

2. The functional device according to claim 1, wherein
the functional part has at least one function selected from an electrode and a sensor.

3. The functional device according to claim 2, wherein
the sensor detects at least one selected from a pressure, a vibration, a blood flow rate, a current, a voltage, an electric field, a magnetic field, a temperature, light, and a chemical quantity.

4. The functional device according to claim 1 or 2, wherein
a thickness of the substrate falls within a range of 0.5 µm or more and 50 µm or less.

5. The functional device according to claim 1 or 2, wherein
a Young's modulus falls within a range of 1 kPa or more and 10 GPa or less.

6. The functional device according to claim 1 or 2, wherein
the blood vessel is a superficial cerebral venous blood vessel, and
the tissue is nervous tissue.

7. The functional device according to claim 1 or 2, wherein
a plurality of through holes each having a diameter falling within a range of 10 µm or more and 1000 µm or less are formed in the substrate.

8. The functional device according to claim 1 or 2, wherein
a plurality of X-ray markers formed of a material that blocks X-rays are formed in the substrate.

9. A guiding catheter that has a cavity inside the guiding catheter and is flexible, wherein
the functional device according to claim 1 or 2 is disposed in the cavity.

10. The guiding catheter according to claim 9, wherein
the functional device is disposed in the cavity in a state of being rounded in a helical shape.

11. A biological interface system, comprising:
a relay that is connected to the functional device according to claim 1 or 2 and is disposed outside the blood vessel;
an external receiving apparatus that is wirelessly connected to the relay and is disposed outside a body; and
an antenna that is connected to the external receiving apparatus and is disposed outside the body.

12. A biological interface system, comprising:
a relay that is connected to the functional device according to claim 1 or 2, is disposed outside the blood vessel, and
includes a light emitting element; and
an external receiving apparatus that is connected to the relay using infrared communication, is disposed outside a body, and includes a light receiving element.

13. The biological interface system according to claim 11 or 12,
wherein
at least one selected from the relay and the external receiving apparatus performs at least one selected from amplifying a signal from the functional device and removing noise from the signal.

14. A method of indwelling a functional device, the method comprising:
a guiding catheter disposing step in which a first end portion of a guiding catheter is disposed at a predetermined position inside a blood vessel; and
a functional device indwelling step in which at the first end portion, a sheet-shaped flexible functional device is discharged from an inside of the guiding catheter into the blood vessel so that the functional device is indwelled inside the blood vessel.

15. The method of indwelling a functional device according to claim 14, wherein
the guiding catheter includes a guidewire, and
in the guiding catheter disposing step, the guiding catheter is guided by the guidewire and is disposed at a predetermined position inside the blood vessel.

16. The method of indwelling a functional device according to claim 14 or 15, wherein
when two end portions of the guiding catheter are defined as the first end portion and a second end portion,
in the functional device indwelling step,
the functional device is inserted into a cavity inside the guiding catheter from the second end portion of the guiding catheter, and
after the functional device is moved to the first end portion of the guiding catheter, the functional device is discharged into the blood vessel.

17. The method of indwelling a functional device according to claim 14 or 15, wherein
in the guiding catheter disposing step,
in a state in which the functional device is disposed at the first end portion in a cavity inside the guiding catheter in advance, the first end portion of the guiding catheter is disposed at a predetermined position inside the blood vessel.
